# EUROPEAN PATENT SPECIFICATION

(11) **EP 0 979 239 B1**
(45) Date of publication and mention of the grant of the patent: **18.10.2006**
(21) Application number: 98910001.1
(22) Date of filing: 13.02.1998
(51) Int. Cl.: C07K 5/00, C07K 14/00, A61K 38/00, A01N 43/04

(54) **PREVENTION AND TREATMENT OF HEPATOCELLULAR CANCER**
VORBEUGUNG UND BEHANDLUNHG DES HEPATOZELLULÄREN KREBSES
PREVENTION ET TRAITEMENT DU CANCER HEPATOCELLULAIRE

(30) Priority: 13.02.1997 US 38375 P
(43) Date of publication of application: 16.02.2000
(73) Proprietor: The Regents of the University of California, Los Angeles, CA 90024-1406 (US)
(72) Inventor: ECONOMOU, James, S., Pacific Palisades, CA 90272 (US); BUTTERFIELD, Lisa, H., Long Beach, CA 90807 (US); RIBAS BRUGUERA, Antoni, Los Angeles, CA 90024 (US)
(74) Representative: W.P. Thompson & Co.
(86) International application number: PCT/US1998/002753
(87) International publication number: WO 1998/035981

(56) References cited:
- EP-A- 0 353 814
- WO-A-96/22787
- US-A- 5 593 972
- US-A- 5 633 234
- DATABASE WPI Section Ch, Week 199008 Derwent Publications Ltd., London, GB; Class B04, AN 1990-053912 XP002206693 & JP 02 005866 A (KWANSAI SHINGIJUTSU), 10 January 1990 (1990-01-10)
- MIZEJEWSKI G J ET AL: "Alpha-fetoprotein derived synthetic peptides: Assay of an estrogen-modifying regulatory segment." MOLECULAR AND CELLULAR ENDOCRINOLOGY, vol. 118, no. 1-2, 1996, pages 15-23, XP001059163 ISSN: 0303-7207
- JOURNAL OF CHROMATOGRAPHY, 1981, Vol. 215, HIRAI et al., "Purification of Specific Antibody to Alpha-Fetoprotein and its Immunological Effect on Cancer Cells", pages 195-210, XP002910064
- GANN., April 1983, Vol. 74, TAGA H., "The Effect of Active Immunization of Rats with Heterologous Alpha-Fetoprotein Upon Hepatocarcinogenesis Induced by 3'-Methyl-4-Dimethylaminoazobenzene", pages 248-257, XP002910065
- TUMOUR BIOLOGY, 1985, Vol. 6, KAMATA Y., "The Prevention of Chemical Hepatocarcinogenesis in Rats by Combining Active and Passive Immunization with Alpha-Fetoprotein", pages 243-256, XP002910066
- CANCER GENE THERAPY, 1996, Vol. 3, No. 3, GALEA-LAURI et al., "Novel Costimulators in the Immune Gene Therapy of Cancer", pages 202-214, XP002910067

### SEQUENCE LISTING

<110> Regents of the University of California, The
<120> Prevention and Treatment of Hepatocellular Cancer
<130> 11969-1EP
<140> 98910001.1
   <141> 1998-02-13
<150> 60/038,375
   <151> 1997-02-13
<150> PCT/US98/02753
   <151> 1998-02-13
<160> 4
<170> PatentIn Ver. 2.1
<210> 1
   <211> 2032
   <212> DNA
   <213> Homo sapiens
<220>
   <221> CDS
   <222> (48)..(1874)
<400> 1
tccatattgt gcttccacca ctgccaataa caaaataact agcaacc atg aag tgg    56
   gtg gaa tca att ttt tta att ttc cta cta aat ttt act gaa tcc aga   104
   aca ctg cat aga aat gaa tat gga ata gct tcc ata ttg gat tct tac   152
   caa tgt act gca gag ata agt tta gct gac ctg gct acc ata ttt ttt   200
   gcc cag ttt gtt caa gaa gcc act tac aag gaa gta agc aaa atg gtg   248
   aaa gat gca ttg act gca att gag aaa ccc act gga gat gaa cag tct     296
   tca ggg tgt tta gaa aac cag cta cct gcc ttt ctg gaa gaa ctt tgc     344
   cat gag aaa gaa att ttg gag aag tac gga cat tca gac tgc tgc agc     392
   caa agt gaa gag gga aga cat aac tgt ttt ctt gca cac aaa aag ccc     440
   act cca gca tcg atc cca ctt ttc caa gtt cca gaa cct gtc aca agc     488
   tgt gaa gca tat gaa gaa gac agg gag aca ttc atg aac aaa ttc att     536
   tat gag ata gca aga agg cat ccc ttc ctg tat gca cct aca att ctt     584
   ctt tgg gct gct cgc tat gac aaa ata att cca tct tgc tgc aaa gct     632
   gaa aat gca gtt gaa tgc ttc caa aca aag gca gca aca gtt aca aaa     680
   gaa tta aga gaa agc agc ttg tta aat caa cat gca tgt gca gta atg     728
   aaa aat ttt ggg acc cga act ttc caa gcc ata act gtt act aaa ctg     776
   agt cag aag ttt acc aaa gtt aat ttt act gaa atc cag aaa cta gtc     824
   ctg gat gtg gcc cat gta cat gag cac tgt tgc aga gga gat gtg ctg      872
   gat tgt ctg cag gat ggg gaa aaa atc atg tcc tac ata tgt tct caa      920
   caa gac act ctg tca aac aaa ata aca gaa tgc tgc aaa ctg acc acg      968
   ctg gaa cgt ggt caa tgt ata att cat gca gaa aat gat gaa aaa cct     1016
   gaa ggt cta tct cca aat cta aac agg ttt tta gga gat aga gat ttt     1064
   aac caa ttt tct tca ggg gaa aaa aat atc ttc ttg gca agt ttt gtt     1112
   cat gaa tat tca aga aga cat cct cag ctt gct gtc tca gta att cta     1160
   aga gtt gct aaa gga tac cag gag tta ttg gag aag tgt ttc cag act     1208
   gaa aac cct ctt gaa tgc caa gat aaa gga gaa gaa gaa tta cag aaa     1256
   tac atc cag gag agc caa gca ttg gca aag cga agc tgc ggc ctc ttc     1304
   cag aaa cta gga gaa tat tac tta caa aat gcg ttt ctc gtt gct tac     1352
   aca aag aaa gcc ccc cag ctg acc tcg tcg gag ctg atg gcc atc acc     1400
   aga aaa atg gca gcc aca gca gcc act tgt tgc caa ctc agt gag gac    1448
   aaa cta ttg gcc tgt ggc gag gga gcg gct gac att att atc gga cac    1496
   tta tgt atc aga cat gaa atg act cca gta aac cct ggt gtt ggc cag    1544
   tgc tgc act tct tca tat gcc aac agg agg cca tgc ttc agc agc ttg    1592
   gtg gtg gat gaa aca tat gtc cct cct gca ttc tct gat gac aag ttc    1640
   att ttc cat aag gat ctg tgc caa gct cag ggt gta gcg ctg caa acg    1688
   atg aag caa gag ttt ctc att aac ctt gtg aag caa aag cca caa ata    1736
   aca gag gaa caa ctt gag gct gtc att gca gat ttc tca ggc ctg ttg    1784
   gag aaa tgc tgc caa ggc cag gaa cag gaa gtc tgc ttt gct gaa gag    1832
   gga caa aaa ctg att tca aaa act cgt gct gct ttg gga gtt            1874
   taaattactt caggggaaga gaagacaaaa cgagtctttc attcggtgtg aacttttctc 1934
   tttaatttta actgatttaa cactttttgt gaattaatga aatgataaag acttttatgt 1994
   gagatttcct tatcacagaa ataaaatatc tccaaatg                         2032
<210> 2
   <211> 609
   <212> PRT
   <213> Homo sapiens
<400> 2
<210> 3
   <211> 9
   <212> PRT
   <213> Homo sapiens
<400> 3
   Gly Val Ala Leu Gln Thr Met Lys Leu
   1 5
<210> 4
   <211> 9
   <212> PRT
   <213> Homo sapiens
<400> 4
   Gly Val Ala Leu Gln Thr Met Lys Val
   1 5

### SEQUENCE LISTING

<110> Regents of the University of California, The
<120> Prevention and Treatment of Hepatocellular Cancer
<130> 11969-1EP
<140> 98910001.1
   <141> 1998-02-13
   <150> 60/038,375
   <151> 1997-02-13
   <150> PCT/US98/02753
   <151> 1998-02-13
<160> 4
<170> PatentIn Ver. 2.1
<210> 1
   <211> 2032
   <212> DNA
   <213> Homo sapiens
<220>
   <221> CDS
   <222> (48)..(1874)
<400> 1
tccatattgt gcttccacca ctgccaataa caaaataact agcaacc atg aag tgg   56
   gtg gaa tca att ttt tta att ttc cta cta aat ttt act gaa tcc aga   104
   aca ctg cat aga aat gaa tat gga ata gct tcc ata ttg gat tct tac   152
   caa tgt act gca gag ata agt tta gct gac ctg gct acc ata ttt ttt   200
   gcc cag ttt gtt caa gaa gcc act tac aag gaa gta agc aaa atg gtg   248
   aaa gat gca ttg act gca att gag aaa ccc act gga gat gaa cag tct    296
   tca ggg tgt tta gaa aac cag cta cct gcc ttt ctg gaa gaa ctt tgc    344
   cat gag aaa gaa att ttg gag aag tac gga cat tca gac tgc tgc agc    392
   caa agt gaa gag gga aga cat aac tgt ttt ctt gca cac aaa aag ccc    440
   act cca gca tcg atc cca ctt ttc caa gtt cca gaa cct gtc aca agc    488
   tgt gaa gca tat gaa gaa gac agg gag aca ttc atg aac aaa ttc att    536
   tat gag ata gca aga agg cat ccc ttc ctg tat gca cct aca att ctt    584
   ctt tgg gct gct cgc tat gac aaa ata att cca tct tgc tgc aaa gct    632
   gaa aat gca gtt gaa tgc ttc caa aca aag gca gca aca gtt aca aaa    680
   gaa tta aga gaa agc agc ttg tta aat caa cat gca tgt gca gta atg    728
   aaa aat ttt ggg acc cga act ttc caa gcc ata act gtt act aaa ctg    776
   agt cag aag ttt acc aaa gtt aat ttt act gaa atc cag aaa cta gtc    824
   ctg gat gtg gcc cat gta cat gag cac tgt tgc aga gga gat gtg ctg    872
   gat tgt ctg cag gat ggg gaa aaa atc atg tcc tac ata tgt tct caa    920
   caa gac act ctg tca aac aaa ata aca gaa tgc tgc aaa ctg acc acg    968
   ctg gaa cgt ggt caa tgt ata att cat gca gaa aat gat gaa aaa cct    1016
   gaa ggt cta tct cca aat cta aac agg ttt tta gga gat aga gat ttt    1064
   aac caa ttt tct tca ggg gaa aaa aat atc ttc ttg gca agt ttt gtt    1112
   cat gaa tat tca aga aga cat cct cag ctt gct gtc tca gta att cta    1160
   aga gtt gct aaa gga tac cag gag tta ttg gag aag tgt ttc cag act    1208
   gaa aac cct ctt gaa tgc caa gat aaa gga gaa gaa gaa tta cag aaa    1256
   tac atc cag gag agc caa gca ttg gca aag cga agc tgc ggc ctc ttc    1304
   cag aaa cta gga gaa tat tac tta caa aat gcg ttt ctc gtt gct tac    1352
   aca aag aaa gcc ccc cag ctg acc tcg tcg gag ctg atg gcc atc acc    1400
   aga aaa atg gca gcc aca gca gcc act tgt tgc caa ctc agt gag gac     1448
   aaa cta ttg gcc tgt ggc gag gga gcg gct gac att att atc gga cac     1496
   tta tgt atc aga cat gaa atg act cca gta aac cct ggt gtt ggc cag     1544
   tgc tgc act tct tca tat gcc aac agg agg cca tgc ttc agc agc ttg     1592
   gtg gtg gat gaa aca tat gtc cct cct gca ttc tct gat gac aag ttc     1640
   att ttc cat aag gat ctg tgc caa gct cag ggt gta gcg ctg caa acg     1688
   atg aag caa gag ttt ctc att aac ctt gtg aag caa aag cca caa ata     1736
   aca gag gaa caa ctt gag gct gtc att gca gat ttc tca ggc ctg ttg     1784
   gag aaa tgc tgc caa ggc cag gaa cag gaa gtc tgc ttt gct gaa gag     1832
   gga caa aaa ctg att tca aaa act cgt gct gct ttg gga gtt             1874
   taaattactt caggggaaga gaagacaaaa cgagtctttc attcggtgtg aacttttctc 1934
   tttaatttta actgatttaa cactttttgt gaattaatga aatgataaag acttttatgt 1994
   gagatttcct tatcacagaa ataaaatatc tccaaatg                         2032
<210> 2
   <211> 609
   <212> PRT
   <213> Homo sapiens
<440> 2
<210> 3
   <211> 9
   <212> PRT
   <213> Homo sapiens
<400> 3
   Gly Val Ala Leu Gln Thr Met Lys Leu
   1 5
<210> 4
   <211> 9
   <212> PRT
   <213> Homo sapiens
<400> 4
   Gly Val Ala Leu Gln Thr Met Lys Val
   1 5

## Description

Primary liver cancer is a major cause of cancer deaths worldwide. Hepatocellular carcinoma (HCC) is the most common type of primary liver cancer, having an incidence of approximately 1.2 million cases per year. In some areas of the world, such as Southeast Asia and South Africa, hepatocellular carcinoma is one of the most common types of malignancies. The high frequency of the diseases appears to be related to the high incidence of hepititis in these regions.

Curative therapy of hepatocellular carcinoma is currently limited to individuals with nonmetastatic disease and involves surgical resection of the tumour with or without liver transplantation. Even surgical resection and transplantation, however, do not cure most tumours because of recurrence after resection. Chemotherapeutic approaches to treatment have, to date, been largely ineffective. There have been no significant advances in the treatment of hepatocellular carcinoma during the last two decades.

Therefore, there remains a need for an effective treatment for hepatocellular carcinoma. The treatment should ideally be suitable for use in lesser developed countries that have the highest incidence of the disease. Further, the treatment should be appropriate for use in individuals with unresectable tumours and with metastatic disease.

EP 0353814 discloses a synthetic peptide endowed with immunological activity, capable of inducing the production of antibodies with high specificity towards alpha-fetoprotein, and their use in the diagnostic field. WO 9622787 discloses methods of inhibiting autoreactive immune cell proliferation in a mammal using alpha-fetoprotein. Mizejewski et al., discloses the estrogen bioassay of a synthetic peptide fashioned after an amino acid sequence from human alpha fetoprotein (HAFP) *(Mizejewski, G. J. et al., (1996) Molecular* and *Cellular Endochrinology, 118, 15-23).* JP 02 005886 discloses a gene containing human alpha fetoprotein domain I having a given amino acid sequence,Hirai et al., discusses the purification of specific antibody to alpha-fetoprotein and its immunological effects on cancer cells. Hirai, et al., (1981) Journal of Chromatography, *215, 195-210).* Kamata et al., illustrates a study on the prevention of chemical heptacarcinogenesis in rats by combining active and passive immunization with Alpha-Petoprotein *(Kamata, Y.et al., (1985) Tumour Biology, 6, 243-256).*

### SUMMARY

In accordance with the present invention, there is provided a composition comprising a peptide consisting of part of an amino acid sequence of an alphafetoprotein molecule or a variant thereof, wherein the part of the alphafetoprotein molecule has an amino acid sequence selected from the group consisting of residues 1-9 of SEQ ID No:2, residues 12-20 of SHQ ID No:2, residues 158-166 of SBQ ID No:2, residues 178-186 of SEQ ID No:2, residues 235-243 of BEQ ID No:2, residues 287-295 of SEQ ID No:2, residues 404-412 of SBCQ ID No:2, residues 441-450 of SEQ ID No:2, residues 492-500 of SBQ IV No:2, residues 542-550 of SBQ ID No:2, residues 547-556 of SEQ ID No:2, and residues 555-563 of SEQ ID No:2, and the variant has an amino acid sequence selected from the group consisting of SEQ ID NO:3 and SBQ ID NO:4.

Therefore, according to the present invention, there is provided a method for preventing or for treating cancer, such as hepatocellular carcinoma, in a mammal, including a human. The method comprises the step of creating an immune response in the mammal to at least part of the amino acid sequence of an alpha-fetoprotein molecule.

The step of creating an immune response can comprise administering to the mammal at least one composition including a peptide comprising at least part of the alpha-fetoprotein amino acid sequence or at least one composition including a peptide comprising at least part of the alpha-fetoprotein amino acid sequence with at least one amino acid substitution. The step of creating an immune response can also comprise administering to the mammal at least one composition including at least part of the cDNA sequence for the alpha-fetoprotein molecule. Further, the step of creating an immune response can comprise administering to the mammal at least one composition including immune system cells transduced with recombinant vector that expresses alpha-fetoprotein cDNA.

According to the present invention, there is provided a composition for immunizing a human to prevent or to treat cancer. The composition can comprise a peptide selected from the group consisting of AFP5, AFP7, AFP13, APP14, AFP18, AFP22, AFP23, AFP28, AFP38, AFP39, AFP45, AFP49, SEQ ID NO: 3 and SEQ ID NO: 4

In accordance with a further aspect of the present invention, there is provided the use of a peptide comprising a part of an amino acid sequence of an alphafetoprotein molecule or a variant thereof, wherein the part of the alphafetoprotein molecule has an amino acid sequence selected from the group consisting of residues 1-9 of SBQ ID No:2, residues 12-20 of SBQ ID No:2, residues 158-166 of SEQ ID No:2, residues 178-186 of SEQ ID No:2, residues 235-243 of 3BQ ID No:2, residues 287-295 of SEQ ID No:2, residues 404-412 of SEQ ID No:2, residues 441-450 of SEQ ID No:2, residues 492-500 of SEQ ID No:2, residues 542-550 of SEQ ID No:2, residues 547-556 of SEQ ID No:2, and residues 555-563 of SEQ ID No:2, and the variant has an amino acid sequence selected from the group consisting of SEQ ID NO:3 and SEQ ID NO:4 for the manufacture of a medicament for treating cancer in a mammal by activating alphafetoprotein peptide specific T lymphocytes to create an immune response against cancer bearing these markers.

In accordance with yet another aspect of the present invention there is provided the use of a cDNA encoding a part of an amino acid sequence of an alphafetoprotein molecule or a variant thereof, wherein the part of the alphafetoprotein molecule has an amino acid sequence selected from the group consisting of residues 1-9 of SEQ ID No:2, residues 12-20 of SEQ ID No:2, residues 158-166 of SEQ ID No:2, residues 178-186 of SBQ ID No:2, residues 235-243 of SEQ ID No:2, residues 287-295 of SBQ ID No:2, residues 404-412 of SEQ ID No:2, residues 441-450 of SBQ ID No:2, residues 492-500 of SEQ ID No:2, residues 542-550 of SEQ ID No:2, residues 547-556 of SEQ ID No:2, and residues 555-563 of SBQ ID No:2, and the variant has an amino acid sequence selected from the group consisting of SBQ ID NO:3 and SBQ ID NO:4 for the manufacture of a medicament for treating cancer in a mammal by activating alphafetoprotein peptide specific T lymphocytes to create an immune response against cancer bearing these markers.

### FIGURES

These and other features, aspects and advantages of the present invention will become better understood with regard to the following description, appended claims, and accompanying figures where:
Figure 1 is a bar graph showing the relative cytotoxicity of CIL generated using human AFP49 peptide;
Figure 2 is a bar graph showing the percent specific lysis versus targets for a standard chromium release assay of CTL generated from peptide-pulsed PBMC from a normal, HLA A2.1 donor, assayed against both peptide targets and AFP targets;
Figure 3 is a plot of mean tumor volume versus days after a turnor challenge of BWIC3, a mAFP-positive murine tumor cell line, for mice immunized with murine AFP cDNA (open boxes), and mice that were not immunized (closed squares);
Figure 4 is a plot of mean tumor volume versus days after a tumor challenge of EI4(parental), a non mAFP-producing murine tumor cell line, for mice immunized with murine AFP cDNA (open boxes) and mice that were not immunized (closed circles);
Figure 5 is a plot of mean tumor volume versus days after a tumor challenge of EL4(AFP), an mAFP-producing murine tumor cell line, for mice immunized with murine AFP cDNA (open boxes), and mice that were not immunized (closed circles);
Figure 6 is a plot of average tumor diameter versus days after a turner challenge of FSA C3H background fibrosarcoma cells stably transfected with AFP-expressing vector or FSA C3H background fibrosarcoma cells stably transfected with the neo-expressing vector only, for mice immunized with plasmid DNA using mouse AFP-AdVShuttle vector neo-containing expression plasmids (closed circles, lower closed squares, closed diamonds) and mice that were not immunized (upper closed squares, closed inverted triangles);
Figure 7 is a plot of average tumor diameter versus days after a tumor challenge of BWIC3 for mice immunized with a plasmid vector that synthesized the mouse AFP gene (closed circles) and mice that were not immunized (closed squares);
Figure 8 is a plot of average tumor diameter versus days after a tumor challenge of BWIC3 for mice that were immunized with a plasmid vector that synthesized the mouse AFP gene (closed triangles, closed diamonds, closed inverted triangles) and mice that were not immunized (closed circles and closed squares);
Figure 9 is an RT-PCR analysis of mRNA isolated from murine DC transduced with AdVmAFP at various multiplicities of infection (MOI), lanes 4-7, compared with various controls, lanes 2, 3, 8 and 9;
Figure 10 is a plot of mean tumor volume versus days after a tumor challenge of EL4(AFP), an mAFP-producing murine tumor cell line, for mice immunized with AdVmAFP transduced dendritic cells (dosed square), mice that were immunized with various control substances (closed upright triangles) and closed inverted triangles) and mice that were not immunized (closed circle); and
Figure 11 is a plot of mean tumor volume versus days after a tumor challenge of BWIC3, an mAFP-producing murine tumor cell line, for mice immunized with AdVmAFP transduced dendritic cells (closed squares) and mice that were not immunized (closed circles).

### DESCRIPTION

The present invention provides a method for preventing or for treating cancer, including hepatocellular carcinoma, in a mammal such as a human where the cancer bears at least a portion of the alphafetoprotein molecule on its surface, by creating an immune response in the mammal to at least part of the alphafetoprotein molecule. The method involves immunizing or genetically manipulating the mammal having the cancer to produce an immune response to at least part of the alphafetoprotein molecule present on the surface of the cancer cells. Then, the immune system of the affected mammal is allowed to destroy the cancer cells bearing the surface marker, thereby preventing a dinical cancer or treating an established cancer.

The majority of human hepatocellular carcinoma cells synthesize human alphafetoprotein (hAFP), a 609 amino acid residue protein, SEQ ID NO:2, which is normally produced by fetal liver cells up until about the time of birth. Hepatocellular carcinoma cells tend to display at least part of the alphafetoprotein molecule on their surface. The presence of alphafetoprotein in hepatocellular carcinoma has been used a marker for screening and diagnostic purposes.

Because alphafetoprotein is normally present during the development of the immune system, it would naturally be assumed that the immune system would not retain the capacity to respond immunologically to the protein. One aspect of the present invention involves the discovery that the immune system of a mammal can be made to respond to alphafetoprotein as a foreign protein and to react to cells having at least part of the alphafetoprotein molecule on its surface as foreign cells. Generating this immune response can, therefore, be used to prevent hepatocellular carcinoma and to treat the disease by causing the mammal's immune system to destroy hepatocellular carcinoma cells.

As disclosed herein, immunization to alphafetoprotein can be accomplished by a variety of means including immunization with synthetic peptides comprising at least part of the alphafetoprotein sequence including synthetic peptides based on at least part of the alphafetoprotein sequence, but have substitutions or other alterations, immunization with at least part of the cDNA sequence for alphafetoprotein thereby causing production and presentation of at least part of the alphafetoprotein molecule to the appropriate immune system cells, the introduction of genetically engineered antigen presenting cells into the mammal, and the use of gene therapy viral vectors to cause the expression of at least part of the alphafetoprotein molecule. The goal of this immunization is to activate alphafetoprotein peptide specific T lymphocytes to create the immune response against cells bearing these surface markers, and preferably thereby to activate cytotoxic T lymphocytes to destroy hepatocellular carcinoma cells.

### 1) DETERMINATION AND PRODUCTION OF HUMAN ALPHAFETOPROTEIN PEPTIDES THAT CREATE AN IMMUNE RESPONSE IN HUMANS

In order to determine if any portion of the human alphafetoprotein molecule is capable of creating an immune response in humans, a series of peptides derived from the whole human alphafetoprotein (hAFP) molecule, SEQ ID NO:2, were tested to determine whether, as class I-restricted peptides, they were capable of generating antitumor responses and capable of serving as target molecules for cytotoxic lymphocytes (CTL). Potentially immunogenic peptides derived from hAFP were selected on the basis of their potential conformity to the HLA A2.1 class I binding grove. HLA A2.1 (HLA A*0201 in the World Health Organization subtype nomenclature) was chosen because it is the most common allele among Caucasians and is also well distributed among other populations. The determination was made as follows.

First, peptide sequences from hAFP, SEQ ID NO:2, were identified that would potentially bind to HLA A2.1 according to published consensus sequences. HLA A2.1 is believed to bind peptides that are eight to ten amino acids in length, but preferably peptides that are nine mers. Amino acids isoleucine, leucine and methionine are believed to be important anchor residues in peptide position 2 and amino acids isoleucine, leucine and valine are believed to be important anchor residues in peptide positions 9 or 10, depending on the peptide length.

Appropriate peptide sequences that conformed to the HLA A2.1 class I binding motif were identified using the University of Wisconsin Genetics Computer Group Program "find patterns" to screen the hAFP sequence, SEQ ID NO:2, and identify nine and ten mer peptides that contained two strong binding "anchor" residues, one at position 2 and one at position 9 or 10 for peptides having nine and ten mers, respectively (designated "strong" peptides); only one strong binding anchor residue (designated "intermediate" peptides); or no strong binding anchor residue (designated "weak" peptides), but having other positive binding residues. Peptide sequences that contained more than one residue thought to abolish binding were eliminated.

The screening study identified a total of seventy-two peptide sequences that potentially conformed to the HLA A2.1 class I binding motif but six of these sequences were eliminated from further consideration because they were difficult to synthesize due to their high hydrophobicity. The remaining sixty-six peptide sequences were synthesized for testing by Chiron Mimetopes (Victoria, Australia) according to techniques known to those with skill in the art. These included ten "strong" peptide sequences, forty-three "intermediate" peptide sequences and thirteen "weak" peptide sequences. Referring now to Table I, there is shown from right to left, respectively, the peptide designation number, the residues of the hAFP sequence, SEQ ID NO:2, represented by the peptide sequence and the amino acids the peptide comprises for each of the sixty-six peptide sequences. The peptide designation number is based on the order of receipt of the peptide from Chiron and is, therefore, nonsequential with

**TABLE I**

| **HUMAN AFP PEPTIDE SEQUENCES** | | |
|---|---|---|
| Peptide Designation Name | Residues in hAFP Protein | Amino Acid Sequence |
| AFP1 | 449-457 | AITRKMAAT |
| AFP2 | 434-442 | AYTKKAPQL |
| AFP3 | 218-226 | LLNQHACAV |
| AFP4 | 257-265 | KLVLDVAHV |
| AFP5 | 158-166 | FMNKFIYEI |
| AFP6 | 135-143 | SIPLFQVPE |
| AFP7 | 12-20 | LLNFTESRT |
| AFP8 | 54-62 | FVQEATYKE |
| AFP9 | 58-66 | ATYKEVSKM |
| AFP10 | 61-69 | KEVSKMVKD |
| AFP11 | 121-129 | RHNCFLAHK |
| AFP12 | 456-464 | ATAATCCQL |
| AFP13 | 404-412 | YIQESQALA |
| AFP14 | 441-450 | QLTSSELMAI |
| AFP15 | 242-250 | KLSQKFTKV |
| AFP16 | 211-219 | KELRESSLL |
| AFP17 | 514-522 | SLVVDETYV |
| AFP18 | 178-186 | ILLWAARYD |
| AFP19 | 187-195 | KIIPSCCKA |
| AFP20 | 270-278 | CRGDVLDCL |
| AFP21 | 291-299 | QQDTLSNKI |
| AFP22 | 547-556 | TMKQEFLINL |
| AFP23 | 555-563 | NLVKQKPQI |
| AFP24 | 570-578 | AVIADFSGL |
| AFP25 | 469-477 | LLACGEGAA |
| AFP26 | 470-478 | LACGEGAAD |
| AFP27 | 438-447 | KAPQLTSSEL |
| AFP28 | 287-295 | YICSQQDTL |
| AFP29 | 300-308 | TECCKLTTL |
| AFP30 | 37-46 | CTAEISLADL |
| AFP31 | 209-218 | VTKELRESSL |
| AFP32 | 284-293 | IMSYICSQQD |
| AFP33 | 232-240 | TRTFQATTV |
| AFP34 | 419-427 | FQKLGEYYL |
| AFP35 | 372-380 | RVAKGYQEL |
| AFP36 | 34-43 | SYQCTAEISL |
| AFP37 | 549-557 | KQEFLINLV |
| AFP38 | 1-9 | MKWVESIFL |
| AFP39 | 492-500 | PVNPGVGQC |
| AFP40 | 476-484 | AADIIIGHL |
| AFP41 | 140-148 | QVPEPVTSC |
| AFP42 | 306-315 | TTLERGQCII |
| AFP43 | 453-461 | KMAATAATC |
| AFP44 | 539-548 | QAQGVALQTM |
| AFP45 | 235-243 | FQAITVTKL |
| AFP46 | 380-388 | LLEKCFQTE |
| AFP47 | 433-441 | VAYTKKAPQ |
| AFP48 | 403-411 | KYIQESQAL |
| AFP49 | 542-550 | GVALQTMKQ |
| AFP50 | 585-593 | GQEQEVCFA |
| AFP51 | 117-126 | SEEGRHNCFL |
| AFP52 | 169-178 | RHPFLYAPTI |
| AFP53 | 253-262 | TEIQKLVLDV |
| AFP54 | 360-369 | RRHPQLAVSV |
| AFP55 | 423-432 | GEYYLQNAFL |
| AFP56 | 507-516 | NRRPCFSSLV |
| AFP57 | 545-554 | LQTMKQEFLI |
| AFP58 | 572-581 | IADFSGLLEK |
| AFP59 | 577-586 | GLLEKCCQGQ |
| AFP60 | 294-302 | TLSNKITEC |
| AFP61 | 278-287 | LQDGEKIMSY |
| AFP62 | 417-425 | GLFQKLGEY |
| AFP63 | 24-33 | NEYGIASILD |
| AFP64 | 65-74 | KMVKDALTAI |
| AFP65 | 350-358 | FLASFVHEY |
| AFP66 | 52-60 | AQFVQEATY |

respect to the amino acid sequence of the hAFP molecule, SEQ ID NO:2.

Next, each of the sixty-six peptides was tested for its ability to bind in a concentration dependent way to HLA A2.1 and, thereby, to stabilize HLA A2.1 in a T2 cell stabilization assay as follows. Each peptide was incubated overnight with TAP1 and TAP2 deleted T2 cells that had been incubated at room temperature the previous night to increase cell surface MHC class I molecule expression. Each peptide was tested for its ability to bind the HLA A2.1 molecule over a range of peptide concentrations, from 0.1 µM-100 µM. In the T2 cell line, only MHC molecules that are filled with eight to ten mer peptides are stable on the cell surface. Stability of HLA A2.1 was assayed by flow cytometry after staining the T2 cells with anti-HLA A2 antibody BB7.2 (ATCC) and goat antimouse-FITC. As positive controls for binding, the FLU matrix peptide (residues 58-66, GILGFVFTL, of FLU matrix 1 protein) and the MART-1 peptide (residues 27-35, AAGIGILTV, of MART-1, GenBank accession no. U06452 for the whole protein ) were used. The FLU matrix peptide consistently stabilized the A2.1 molecules on T2 cells at concentrations of 0.5µM.

Referring now to Table II, there is shown a list of twenty-two of the sixty-six hAFP peptides. Column 1 lists the peptide designation number, column two identifies the residues of the hAFP sequence, SEQ ID NO:2, represented by the peptide sequence, and column three identifies the number of anchor residues within the sequence.

Column four of Table II lists the minimum concentration of peptide required to bind HLA 2.1 on T2 cells. As can be seen, six of the ten "strong" peptide sequences and seven of the forty-three "intermediate'' peptide sequences showed binding ability to HLA 2.1. Further, none of the thirteen "weak" peptide sequences showed binding ability to HLA A2.1.

Further, each of the sixty-six peptides was also tested for their rate of dissociation from class I molecules over time in an EBV lymphoblastoid cell off-kinetics assay, because it has been found that the off-kinetics, that is the dissociation rate, of a peptide bound to a class I molecule is significantly predictive of the immunogenicity of that peptide. For example, for non-self binding peptides such as viral peptides HPV 16 E7, EBV LMP2, FLU M1 and HTV pol., it has been found that the strongest binding peptides showing the slowest off-kinetics were the most immunogenic. Further, it has been found that a number of known self-protein, immunogenic epitopes from melanoma antigens such as gp 100, MART-1, had one anchor reside and less stable binding affinity by a soluble class I reconstitution assay, but very slow off kinetics. See, for example, Bakker, A.B., et al.,

### Analogues of CTL epitopes

**TABLE II**

| **HUMAN AFP PEPTIDE SEQUENCES & BINDING CHARACTERISTICS** | | | | |
|---|---|---|---|---|
| Peptide Designation Name | Residues in hAFP Protein | Number of Anchor Residues | Minimum Concentration of Peptide to Stabilize A2 on T2 cells | Time of Peptide Stability on Lymphoblastoid Cells |
| AFP3 | 218-216 | 2 | 0.5µM | (n.s.) (not stable) |
| AFP4 | 257-265 | 2 | 0.5µM | (n.s.) |
| AFP5 | 158-166 | 2 | 0.1µ | > 6 hrs |
| AFP6 | 135-143 | 1 | (n.s.) (not stable) | > 6 hrs |
| AFP7 | 12-20 | 1 | 50 µM | > 6 hrs |
| AFP8 | 54-62 | 1 | (n.s.) | > 2 hrs |
| AFP13 | 404-412 | 1 | 10.0µM | > 2 hrs |
| AFP14 | 441-450 (10 mer) | 2 | 50.0µM | > 2 hrs |
| AFP16 | 211-219 | 1 | (n.s.) | 2 hrs |
| AFP17 | 514-522 | 2 | 1.0µM | (n.s.) |
| AFP18 | 178-186 | 1 | (n.s.) | > 6 hrs |
| AFP22 | 547-56 (10 mer) | 2 | (n.s.) | 4 hrs |
| AFP23 | 555-563 | 1 | (n.s.) | > 6 hrs |
| AFP28 | 287-295 | 1 | 50µM | 2 hrs |
| AFP34 | 419-427 | 1 | 100µM | (n.s.) |
| AFP37 | 549-557 | 1 | (n.s.) | < 2 hrs |
| AFP38 | 1-9 | 1 | 0.5µM | < 2 hrs |
| AFP39 | 492-500 | 0 | (n.s.) | > 6 hrs |
| AFP45 | 235-243 | 1 | 100µM | > 6 hrs |
| AFP49 | 542-550 | 1 | (n.s.) | > 6 hrs |
| AFP60 | 294-302 | 1 | 50µM | (n.s.) |
| AFP64 | 65-74 (10 mer) | 2 | 100µM | (n.s.) |
| CONTROLS: | | | | |
| | | | | |
| MART-1 peptide | Residues 27-35 of MART-1 | 1 | 50µM | 2 hrs |
| | | | | |
| FLU matrix peptide | Residues 58-66 of FLU matrix 1 protein | 2 | 0.5µM | > 6 hrs |

*with improved MHC class-I binding capacity elicit anti-melanoma CTL recognizing the wild-type epitope.* Int J Cancer, 1997. 70(3): p. 302-9; and van der Burg, S.H., et al., *Do epitopes derived from autoantigens display low affinity for MHC class I? (letter).* Immunol Today, 1997. 1892): p. 97-98; each incorporated herein by reference in their entirety.

The EBV lymphoblastoid cell off-kinetics assay was performed as disclosed in van der Burg, S.H., et al., *Immunogenicity of peptides bound to MHC class I molecules depends on the MHC-peptide complex stability.* J. Immunology, 1996. 156(9): p.3308-3314, incorporated herein by reference in its entirety. Briefly, HLA A2.1 EBV lymphoblastoid cells were stripped of surface class I peptides and β2 microglobulin in a mild pH 3.2 acid buffer which renders MHC molecules unstable. Each peptide was immediately pulsed onto the stripped cells in excess at 200 µM for 1 hour in the presence of β2 microglobulin. Excess unbound peptide was then washed off and the cells were incubated at 37°C and followed for 0, 2, 4 and 6 hours. The cells were washed at the end of each time point and stained for HLA A2 with the BB7.2 antibody. The peptide-class I complex was considered stable if the mean fluorescence intensity increased at least 1.5-fold from cells that were stripped but not pulsed with peptide.

Both the T2 cell stabilization assay and the EBV lymphoblastoid cell off-kinetics assay were performed for each peptide at least twice. Referring again to Table II, column 5 shows the time of peptide stability on the EBV lymphoblastoid cells. As can be seen, only three of the strong peptides (AFP5, AFP14 and AFP22), twelve of the forty-three intermediate peptides (including AFP49) and one of the weak peptides showed a level of slow off-kinetics. Taking into consideration both the T2 cell stabilization assay and the EBV lymphoblastoid cell off-kinetics assay, it can be seen that seven of the peptide sequences that gave the best results in both assays were AFP5, AFP7, AFP13, AFP14, AFP28, AFP38 and AFP45.

Next, the peptides listed in column 1 of Table III were then used to generate peptide specific CTL *in vitro* by the method disclosed in Plebanski, M. et al., *Induction of peptide-specific primary cytotoxic T lymphocyte responses from human peripheral blood.* Eur J. Immunol. 1995. 25(6): p. 1783-7 and the CTL were tested for their ability to lyse A2.1-positive, AFP-positive hepatocellular carcinoma cells. Lysis would suggest that the peptide is a naturally processed, immunogenic epitope of human AFP and potentially a target antigen. HLA A2.1 donors and cell lines were screened with the BB7.2 (HLA A2) antibody (ATCC) and confirmed and subtyped by PCR and direct sequence analysis by the UCLA Tissue Typing

**TABLE III**

| **HUMAN AFP PEPTIDE CYTOTOXICITY** | | | | | |
|---|---|---|---|---|---|
| Peptide Designation Number | CTL Culture CD4/CD8 Phenotype | T2+Specific Peptide Cytotoxicity | | AFP+/HLA A2.1 + HepG2 Cytotoxicity | |
| | | 50:1 | 74% | 20:1 | 7% |
| AFP3 | CD8+ | 10:1 | 28% | 5:1 | 4% |
| | | 50:1 | 94% | 20:1 | 7% |
| AFP4 | CD8+ | 10:1 | 15% | 5:1 | 4% |
| | | 50:1 | 75% | 20:1 | 7% |
| AFP5 | CD8+ | 10:1 | 41% | 5:1 | 4% |
| | | 50:1 | 52% | | |
| AFP6 | CD8+ | 10:1 | 17% | (n.t.) | |
| | | 50:1 | 22% | 50:1 | 15% |
| AFP7 | CD8+ | 10:1 | 0 | 10:1 | 6% |
| | | 20:1 | 56% | 20:1 | 24% |
| AFP22 | CD8+/CD4+ | 5:1 | 13% | 5:1 | 9% |
| | | 20:1 | 45% | 20:1 | 15% |
| AFP23 | CD8+/CD4+ | 5:1 | 10% | 5:1 | 2% |
| | | 25:1 | 32% | 25:1 | 2% |
| AFP37 | CD8+/CD4+ | 5:1 | 16% | 5:1 | 0 |
| | | 50:1 | 54% | 50:1 | 12% |
| AFP38 | CD8+/CD4+ | 10:1 | 18% | 10:1 | 0 |
| | | 50:1 | 54% | 50:1 | 29% |
| AFP39 | CD8+/CD4+ | 10:1 | 18% | 10:1 | 12% |
| | | 50:1 | 25% | 50:1 | 27% |
| AFP45 | CD8+/CD4+ | 10:1 | 14% | 10:1 | 9% |
| | | 50:1 | 46% | 50:1 | 45% |
| AFP49 | CD8+/CD4+ | 10:1 | 23% | 10:1 | 17% |
| | | 50:1 | 100% | | |
| Residues 58-66 of FLU | CD8+ | 10:1 | 45% | n.t. | |
| | | 50:1 | 100% | | |
| Residues 27-35 of MART-1 | CD8+ | 10:1 | 80% | n.t. | |

Laboratory, according to techniques known to those with skill in the art. Briefly, peptide specific CTL were generated to the peptides listed in Table III AFP peptides as follows. 2x10⁷ peripheral blood mononuclear cells (PBMC) from a normal A2.1 donor were purified by Ficoll gradient. These PBMC were pulsed with 50µg/ml peptide in 1 ml serum-free media for 90 minutes at 37°C. The cells were then rinsed once and placed in a 24-well plate at 3 x 10⁶ PBMC in 1.5 ml of 10% autologous serum RPMI medium per well on day 0 with IL-7 (10 ng/ml) and KLH (4.5 µg/ml) in RPMI/10% autologous serum. CTL were restimulated weekly by removing the non-adherent cells and adding them to fresh, peptide-pulsed, washed and irradiated PBMC at 1:1 PBMC to CTL ratio. IL-2 was added twice weekly at 10 units/ml.

After three weeks of culture, the putative hAFP pepdde-generated CTL were tested for cytotoxicity in a standard 4 hour ⁵¹Cr-release assay. The CTL were tested for peptide-specific killing against T2 cells pulsed with the specific hAFP peptide used to generate the CTL and compared with T2 cells pulsed with the FLU matrix peptide or the MART-1 peptide as controls. Non-specific NK killing was assessed with the NK sensitive target K562. The CTL were also tested against the HLA A2.1-positive, AFP-positive human hepatocellular carcinoma cell line, HepG2.

Referring now to Table III, there are shown the cytotoxicity results of tests for the twelve AFP peptide sequences used to generate CTL from normal donors that gave positive peptide cytotoxicity results. Column 2 shows the CD4/CD8 phenotype of the bulk lymphocyte culture. Columns 3 and 4 show, respectively, the levels of cytotoxicity against peptide-pulsed T2 cells and HepG2 targets with their effector (CTL) to target ratio (E:T).

As can be seen in Table III, peptides AFP22, AFP39, AFP45 and AFP49 demonstrated high levels of specific killing of AFP+, HLA A2.1 + HepG2 cells. It can be noted that AFP22 and AFP49 have a four amino acid overlap, residues 547-550 of hAFP SEQ ID NO:2. Further, AFP22 has a two amino acid overlap, residues 555-556 of SEQ ID NO:2, with AFP23, which showed marginal HepG2 killing.

CTL generated using AFP49 were retested and the HepG2 cytotoxicity was maintained. Further, new AFP49 peptide-generated CTL cultures were made using two different, normal HLA A2.1 donors. Additional targets were used to confirm that the cytotoxicity observed using AFP49 was AFP antigen-specific and class I restricted.

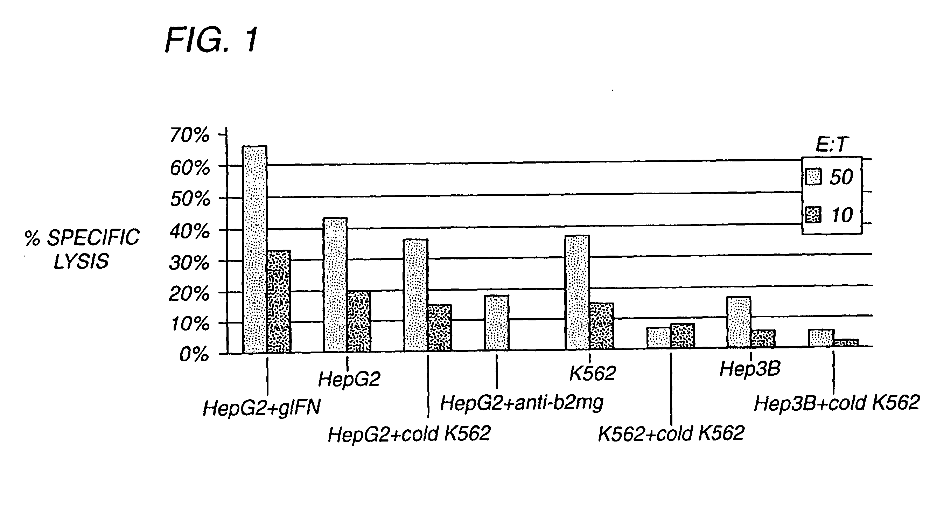

Referring now to Figure 1, there is shown representative data of these tests. First, to confirm that the observed cytotoxicity was class I restricted, anti-β2 microglobulin antibody was used to block the CTL-T cell receptor interaction on HepG2 cells. This resulted in a significant reduction in HepG2 lysis. Next, to eliminate non-specific NK/LAK killing, a 40-fold excess of unlabeled (cold) K562 cells was added. This did not result is a significant reduction in HepG2 lysis. Further, MHC class I expression was upregulated on HepG2 cells by overnight incubation with γIFN (50 units/ml). As can be seen, MHC class I expression upregulation increased HepG2 lysis. Also, an AFP+, HLA A2.1-negative hepatocellular carcinoma cell line, Hep3B, that is, a class I-mismatched hepatocellular carcinoma cell line, was also used as a target. AFP49 CTL lysed these Hep3B targets at a very low level. This small amount of observed Hep3B lysis was eliminated by adding an excess of cold K562 cells, in contrast to the retention of specific killing of HepG2 when cold K562 cells were added.

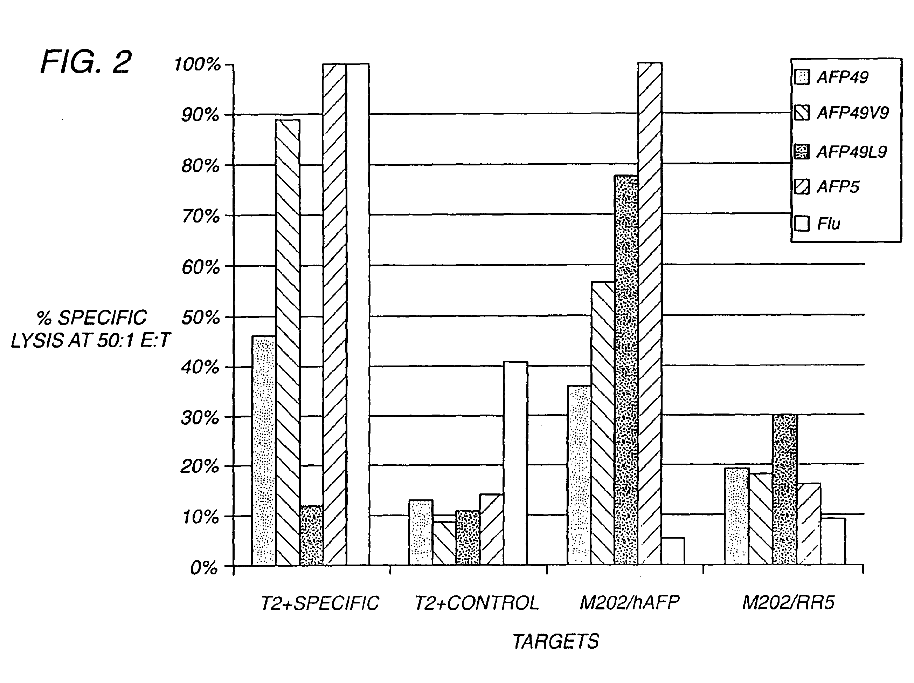

Referring now to Figure 2, there is shown a bar graph of percent specific lysis versus targets for a standard chromium release assay of CTL generated from peptide-pulsed PBMC from a normal, HLA A2.1 donor assayed against both peptide targets and AFP targets. To confirm the peptide specificity of the CTL, each culture was tested against T2 cells pulsed with the specific peptide from which the CTL culture was made, left most bars, and compared with T2 cells pulsed with a different HLA A2.1 binding peptide as a control, second group of bars from the left. As can be seen, the AFP49 peptide culture, the AFP49V9 peptide culture, the AFPS peptide culture and the control FLU matrix peptide culture all showed peptide specificity by lysis of T2 cells pulsed with the specific peptide, but not against T2 cells pulsed with a different peptide.

Referring again to Figure 2, each of these peptide-specific CTL cultures was also tested for killing of M202 (HLA A2.1 +/AFP-) melanoma cells transduced with either AdVhAFP or the control AdVRR5. Each peptide-specific CTL culture of the AFP peptides AFP5 and AFP49, as well as the peptides AFP49L9, SEQ ID NO:3 (GVALQTMKL) and AFP49V9, SEQ ID NO:4 (GVALQTMKV), which have a single amino acid substitution in position 9 of AFP49, show significantly more killing of M202 cells transduced with AdVhAFP than killing of M202 cells transduced with the control RR5. The FLU peptide-specific CTL cultures killed both M202/AdVhAFP and M202/RR5 with similar, background levels of cytotoxicity.

M202 cells are known to correctly process and present the HLA A2.1-restricted, immunodominant MART-1 peptide. Therefore, they are an ideal cell line to transduce with AdVhAFP and expect that the correct HLA A2.1-restricted epitopes from AFP will be processed and presented on the surface. Hence, this experiment demonstrates that AFP5, AFP49, AFP49L9, SEQ ID NO:3 and AFP49V9, SEQ ID NO:4 are naturally processed and presented peptides that can be used to target CTL to kill AFP+ tumors. Further, as can be seen AFP49L9, SEQ ID NO:3, and AFP49V9, SEQ ID NO:4, peptide-specific CTL cultures kill M202/AdVhAFP even more effectively than AFP49 peptide-specific CTL cultures and, therefore, AFP49L9, SEQ ID NO:3, and AFP49V9, SEQ ID NO:4, are improved peptides for targeting the immune response to AFP+ cells.

Thus, as can be appreciated from this disclosure, the present invention includes preventing or treating a cancer in a mammal, including a human, where the cancer cells bear at least part of the alphafetoprotein molecule as a surface marker. The prevention or treatment is accomplished by administering to the mammal a composition including a peptide that comprises at least part of the alphafetoprotein molecule or a peptide that has been produced by substitution of or other alterations to at least part of the alphafetoprotein molecule. These peptides include AFP5, AFP7, AFP13, AFP14, AFP18, AFP22, AFP23, AFP28 AFP38, AFP39, AFP45, AFP49, AFP49L9, SEQ ID NO:3, and AFP49V9, SEQ m NO:4.

### 2) IMMUNIZATION OF MAMMALS USING ALPHAFETOPROTEIN cDNA TO CREATE AN IMMUNE RESPONSE TO CELLS BEARING ALPHAFETOPROTEIN ON THEIR SURFACE, INCLUDING HEPATOCELLULAR CANCER CELLS

Immunizing mammals with alphafetaprotein cDNA creates an immune response that is partially or fully protective against challenges with tumor cells bearing alphafetoprotein on their surface, including hepatocellular cancer cells. This effect was demonstrated as follows:

Human alphafetoprotein cDNA was produced as follows. First, human alphafetoprotein cDNA was generated by PCR techniques from total RNA made from Hep3B cells (available from ATCC) by the Trizol method (Life Technologies, Gaithersburg, MD) according to the manufacture's instructions) and by the RNAzolB method (TelTest, Friendswood, TX). Approximately 1 µg of total RNA was used in an RT-PCR reaction using the Perkin Elmer RT-PCR kit and AFP-specific primers based on the published sequence. The 5' primer was 5' GCA ACC ATG AAG TGG GT. The 3' primer was 5' AAC TCC CAA AGC AGC ACG AGT. The primers included the entire coding region (ATG to the stop codon) with a restriction endonuclease site XbaI incorporated into the primer, and ending with six bases (CTC TCT) to facilitate enzyme cleavage after PCR. Primer sequences were synthesized by Operon Technologies, 50 nM scale, unpurified.

The human alphafetoprotein PCR cDNA products produced above were analyzed on an agarose gel to check their size. Correctly sized products were purified on a Qiagen PCR-quick clean-up column, digested with the XbaI enzyme whose site was designed into the primers, and used in a cloning reaction into either pRcCMV (for human) or pCR3.1 (for murine) mammalian expression vectors (Invitrogen, Carlsbad, CA) according to techniques known to those with skill in the art. Positive plasmids were identified by miniprep analysis. These positive plasmids were maxiprepped and an aliquot was sequenced by the DNA sequencing core facility at UCLA to confirm the sequence identity of the inserts. The sequence data was for one strand only, and confirmed the identity of the AFP inserts. Therefore, the human AFP cDNA cloned was identical to the human AFP published sequence, GenBank accession Nos J00077, J00076, V01514, bases 48-1877, SEQ ID NO:1.

Murine AFP cDNA (mAFP cDNA) was cloned using corresponding methods to the methods disclosed above used to clone human AFP cDNA, but with mouse-specific primers. The 5' murine specific primers was 5' GCC ATG AAG TGG ATC ACA. The 3' murine specific primer was TTA AAC GCC CAA AGC ATC A. The mouse AFP-positive cell line used to isolate total RNA was Hepa16. All stable transfectants and intramuscular injection experiments disclosed herein were performed with cDNA clones containing the signal-sequence.

Next, the mAFP cDNA was placed in the eucaryotic expression vector VR1012 (Vical, Inc., San Diego, CA), The VR1012 expression vector contains the strong constitutive CMV immediate early promoter/enhancer, including an intron for enhanced expression, a BGH termination and poly A sequences for *in vivo* expression.

C57BL/6 mice were given im injections of 100µg VR1012 containing the mAFP cDNA or saline as a control once a week for three weeks. One week after the last injection, both the VR1012 mAFP cDNA immunized mice and the unimmunized group of control mice were challenged with 4x10⁶ viable BWIC3 hepatocellular carcinoma cells obtained from a single cell suspension of progressively growing tumors in syngeneic mice. BWIC3 is a mAFP-positive murine cell line.

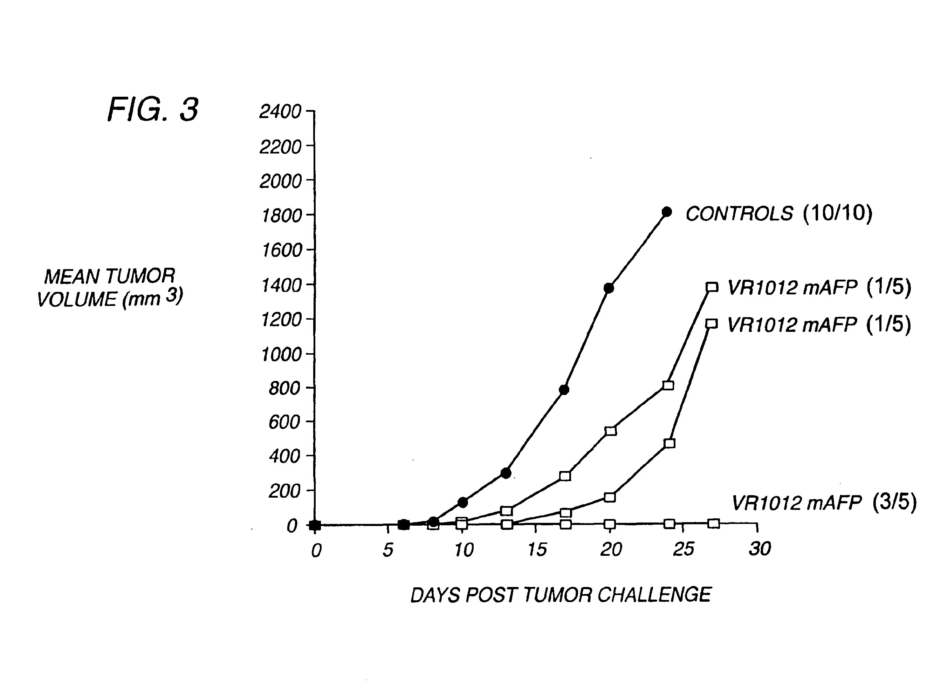

Referring now to Figure 3, it can be seen that immunized animals (open boxes), showed a delayed tumor growth or complete protection compared with control animals (closed squares). These finding were replicated several times. In a corresponding experiment, im injections of a plasmid vector expressing the MART-1 melanoma antigen did not protect animals from a BWIC3 hepatocellular carcinoma cell challenge (data not shown).

In another group of experiments, a surrogate murine hepatocellular carcinoma line was constructed by stably transfecting the EL4 (H-2^{b}) lymphoma with mAFP cDNA. The tumor line EL4(mAFP) has the same *in vivo* growth kinetics as the parental EL4 cell line. Using RT-PCR, it appears that the EL4(mAFP) tumor cell line produces 1 % or less of the levels of AFP as BWIC3 hepatocellular carcinoma cell line.

C57BL/6 mice were given im injections of 100µg VR1012 containing the mAFP cDNA or saline as a control once a week for three weeks. One week after the last injection, both the VR1012 mAFP cDNA immunized mice and the unimmunized group of control mice were challenged with 7.5x10⁵ viable EL4(parental) or EL4(mAFP) cells.

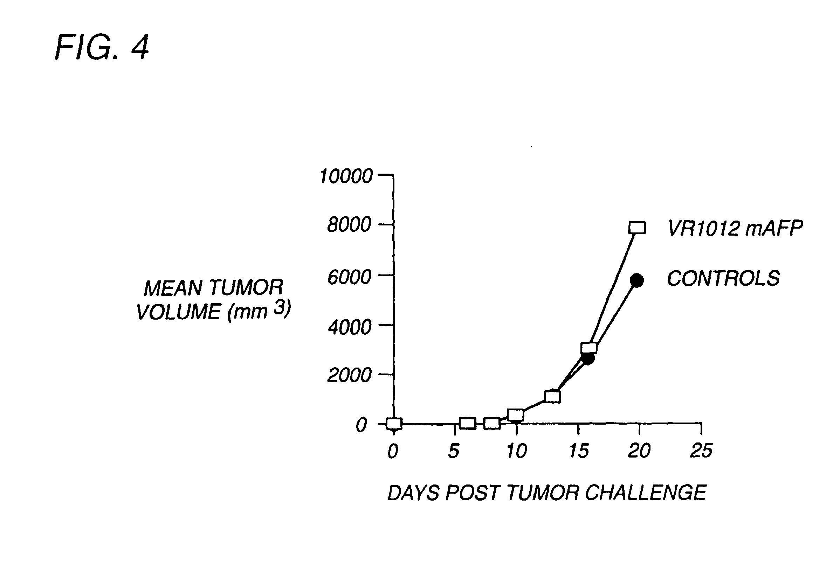

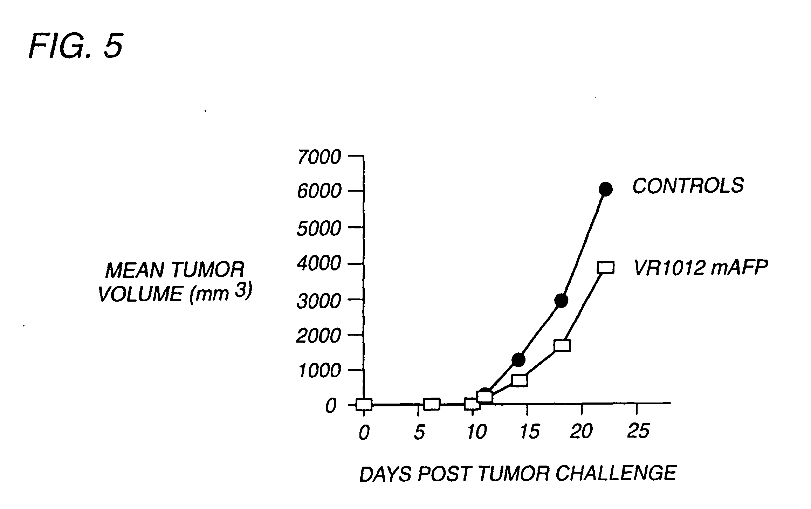

Referring now to Figure 4, it can be seen that immunized animals (open boxes), and control animals (closed circles) showed no difference when challenged with EL4(parental) cells (p=0.07, student's T test). However, as can be seen in Figure 5, immunized animals (open boxes), did show partial protection compared with control animals (closed circles), when challenged with EL4(mAFP) cells (p=0.07, student's T test). These findings were also replicated several times.

In an additional series of experiments, protection against challenges with cells bearing alphafetoprotein on their surface was demonstrated using stably-transfected mouse fibrosarcoma cell lines as a surrogate. First, stably-transfected mouse fibrosarcoma cell lines were produced by either the DOTAP lipofection method (Boehringer Mannheim) according to the manufacture's instruction) and a CaPO₄ precipitation method (according to techniques well known to those with skill in the art). In summary, the DOTAP lipofection method used 1 x 10⁵ cells per well in a 6-well plate, adhered overnight the previous night. 2.5 ug plasmid (murine AFP pCR3.1) was mixed in 25 µl of 20 mM Hepes and 15 µl lipid in 50 µl Hepes at room temperature for 15 min. This was diluted into 1 ml of culture medium (RPMI1640 containing 10% fetal calf serum and antibiotics), and added to the cells in the wells. After 4-6 hours, the solution was replaced with 2 ml fresh culture medium. After 48-72 hours, selection was started with G418 (geneticin) @ 500 µg/ml (total concentration, 75 % active). After 2-3 weeks of selection, any potential transfectants were tested by RT-PCR for expression of mouse AFP RNA, neo-RNA and semi-quantified with murine APRT gene expression.

The effectiveness of AFP immunization in preventing tumor production in mammals was demonstrated as follows. Mouse AFP-pCR3.1 plasmid and mouse AFP-AdVShuttle vector plasmid (pLpA CMV) were prepared according to techniques known to those with skill in the art, and mouse AFP-Vical vector VR1012 was constructed. Murine fibrosarcoma cell lines FSA, NFSA, MCAK and SVEC were stably transfected with mAFP PCR3.1, as above.

C3H mice were immunized by weekly intramuscular injections for three weeks of plasmid DNA using mouse AFP-AdVShuttle vector neo-containing expression plasmids prepared endotoxin-free with a Qiagen plasmid prep kit (50 µg plasmid in 50 µl PBS). The C3H mice were then challenged with FSA C3H background fibrosarcoma cells stably transfected with AFP-expressing vector or FSA C3H background fibrosarcoma cells stably transfected with the neo-expressing vector only, to determine whether an AFP anti-self antigen response could be generated or whether use of stable transfectants expressing neomycin would create an anti-neo (non-self antigen) response that would mask the AFP response. Tumor cells were passaged in vivo, and a single cell suspension was used for tumor challenges.

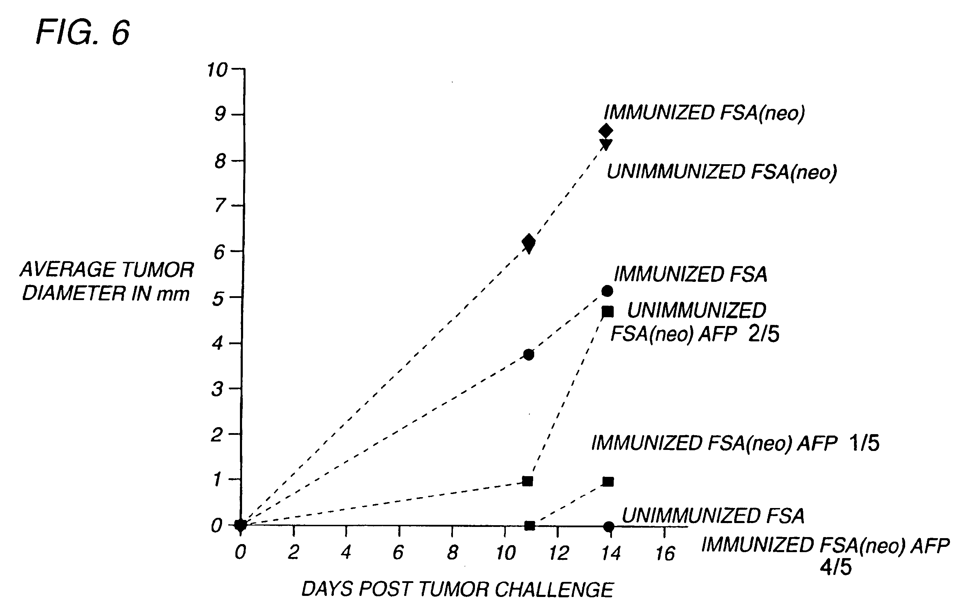

Referring now to Figure 6, it can be seen that by day 18 post-tumor challenge, only one of the immunized CH3 mice (lower closed squares) challenged with FSA C3H background fibrosarcoma cells stably transfected with AFP-expressing vector showed any tumor growth (a 3 mm x 3 mm tumor), while the remaining four immunized CH3 mice (closed circles) challenged with FSA C3H background fibrosarcoma cells stably transfected with AFP-expressing vector did not show any sign of tumor growth. By contrast, two of the five unimmunized C3H mice (upper closed squares) challenged with FSA C3H background fibrosarcoma cells stably transfected with AFP-expressing vector showed any tumor growth (mean 6.8 mm²). FSA parental tumor cells and neo-vector-FSA cells grew similarly in both immunized (closed diamonds) and unimmunized (closed inverted triangles) CH3 mice. This protocol was repeated and similar results were obtained. (Data not shown.)

A second experiment was performed using C57L/J ("leaden") mice from Jackson Labs (Bar Harbor Maine). These mice were immunized with a plasmid vector from Vical (VR1012) that does not contain neomycin and, therefore, synthesized only the mouse AFP gene. The C57L/J mice were challenged with a murine syngeneic tumor cell line, BWIC3 from ATCC. These BWIC3 cells synthesize a much higher level of mouse AFP than the stably-transfected murine fibrosarcoma cells produced as disclosed above.

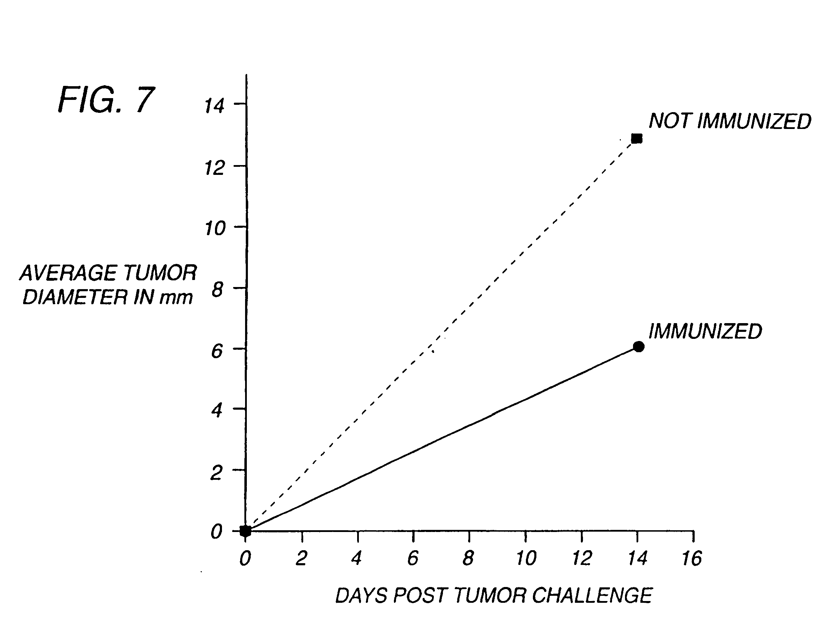

The C57L/J mice were immunized as described above using mAFP-Vical vector and a tumor challenge of 1 x 10⁶ BWIC3 cells per mouse was made subcutaneously. Referring now to Figure 7, it can be seen that at day 14 post-tumor challenge, unimmunized C57L/J mice (closed squares) had tumors that averaged two times larger than the tumors in immunized C57L/J mice (closed circles).

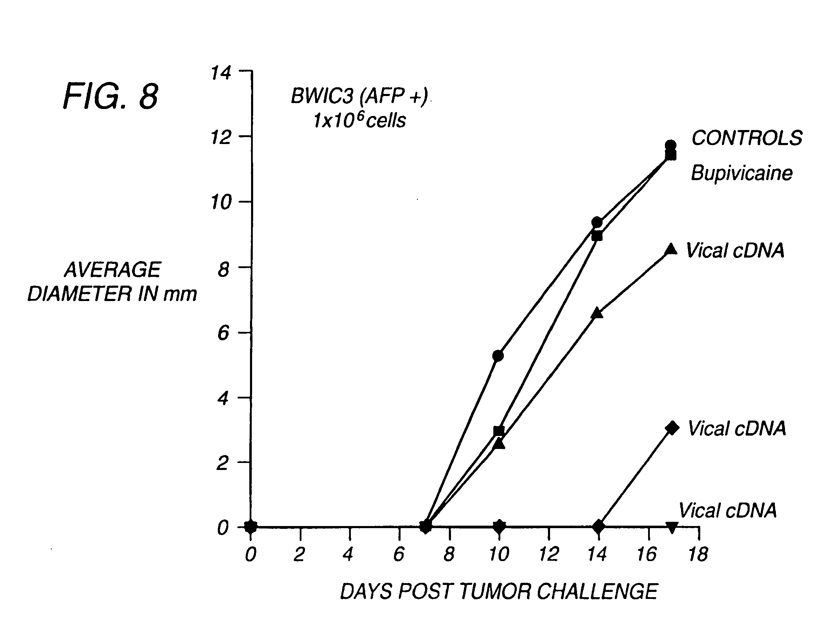

In a third experiment, additional C57L/J mice were immunized with a plasmid vector (VR1012) that synthesized only the mouse AFP gene and challenged with 1 x 10⁶ BWIC3 cells as disclosed above. Referring now to Figure 8, it can be seen that by seventeen days post challenge, all five unimmunized (closed circles and closed squares) mice had tumors, averaging 11.4 mm² in diameter. By contrast, three of the five mice immunized (closed triangles) with mAFP-Vical had tumors averaging 9 mm², one immunized mouse (closed diamonds) had a small 3 mm² tumor, and one mouse (closed inverted triangles) did not show any tumor.

Therefore, as can be appreciated from this disclosure, the present invention includes preventing or treating a cancer in a mammal, including a human, where the cancer cells bear at least part of the alphafetoprotein molecule as a surface marker. The prevention or treatment is accomplished by administering to the mammal a composition including at least a portion of the alphafetoprotein cDNA to create an immune response against at least part of the alphafetoprotein molecule.

### 3) IMMUNIZATION OF MAMMALS USING GENETICALLY-ENGINEERED DENDRITIC CELLS TO CELLS BEARING ALPHAFETOPROTEIN ON THEIR SURFACE, INCLUDING HEPATOCELLULAR CANCER CELLS

Immunizing mammals with dendritic cells transduced with a recombinant adenovirus vector that expresses murine AFP (AdVmAFP) alphafetoprotein cDNA creates an immune response that is partially or fully protective against challenges with hepatocellular cancer cells. This effect was demonstrated as follows:
First, a recombinant adenovirus vector that expresses murine AFP (AdVmAFP) was constructed, according to techniques known to those with skill in the art. See for example, Ribas, A., L. H. Butterfield, W. H. McBride, S. M. Jilani, L. A. Bui, C. M. Vollmer, R. Lau, V. B. Dissette, B. Hu, A. Y. Chen, J. A. Glaspy, and J. S. Economou. 1997. Genetic immunization for the melanoma antigen MART-1/Melan-A using recombinant adenovirus-transduced murine dendritic cells. *Cancer Res 57:2865*; and Toloza, E. M., K. Hunt, S. Swisher, W. McBride, R. Lau, S. Pang, K. Rhoades, T. Drake, A. Belldegrun, J. Glaspy, and J. S. Economou. 1996. In vivo cancer gene therapy with a recombinant interleukin-2 adenovirus vector. *Cancer Gene Ther 3:11,* incorporated herein by reference in its entirety. Then, dendritic cells were generated from C57BL/6 bone marrow differentiated for seven days in GM-CSF and IL-4, according to techniques known to those with skill in the art. See for example, Ribas, A., L. H. Butterfield, W. H. McBride, S. M. Jilani, L. A. Bui, C. M. Vollmer, R. Lau, V. B. Dissette, B. Hu, A. Y. Chen, J. A. Glaspy, and J. S. Economou. 1997. Genetic immunization for the melanoma antigen MART-1/Melan-A using recombinant adenovirus-transduced murine dendritic cells. *Cancer Res 57:2865;* and Inaba, K., M. Inaba, N. Romani, H. Aya, M. Deguchi, S. Ikehara, S. Muramatsu, and R. M. Steinman. 1992. Generation of large numbers of dendritic cells from mouse bone marrow cultures supplemented with granulocyte/macrophage colony-stimulating factor. *J Exp Med 176:1693,* incorporated herein by reference in their entirety.

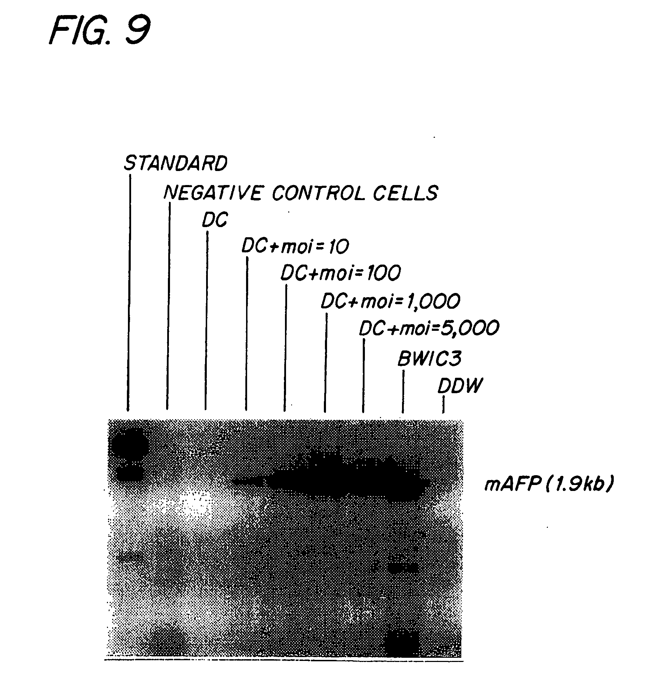

Referring now to Figure 9, there is shown an RT-PCR analysis of mRNA isolated from murine DC transduced with AdVmAFP at various multiplicities of infection (MOI). Reading from left to right, lane 1 shows gel size standards; lane 2 shows the results for mAFP negative cells used as a negative control; lane 3 shows the results for murine dendritic cells used as a negative control; lanes 4-7 show the results for murine dendritic cells transduced with AdVmAFP at a MOI of 10, 100, 1,000 and 5,000, respectively; lane 8 shows the results for BWIC3 cells used as a positive control (upper most line at approximately 1.9 kb); and lane 9 shows the results for double distilled water (DDW), as a no-template control for PCR contamination. As can be seen, the recombinant adenovirus vector that expresses murine AFP (AdVmAFP) successfully transduced the dendritic cells.

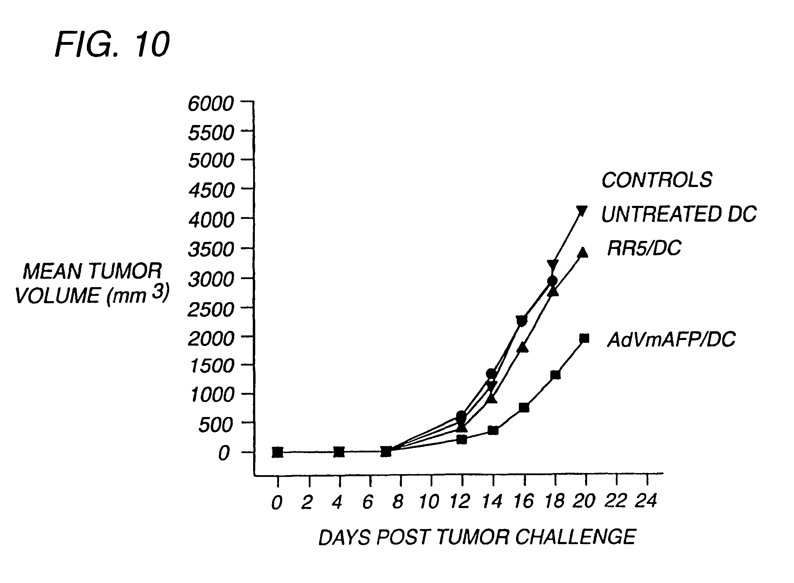

Next, three groups of five C57BL/6 mice were prepared by giving one iv injection per week for two weeks of either 5x10⁵ dendritic cells transduced at an MOI of 100 with AdVmAFP, RR5 (an empty El-deleted adenovirus), or untreated dendritic cells. These three groups of mice and one group of uninjected mice serving as a control were challenged with 7.5x10⁵ EL4(AFP) one week after the last injection. The results are shown in Figure 10. As can be seen, neither the mice injected with RRS (closed upright triangles), or untreated dendritic cells (closed inverted triangles), nor the control mice (closed circles), showed protection against the tumor challenge. By contrast, mice injected with 5x10⁵ dendritic cells transduced at an MOI of 100 with AdVmAFP (closed squares), showed partial protection against the tumor challenge.

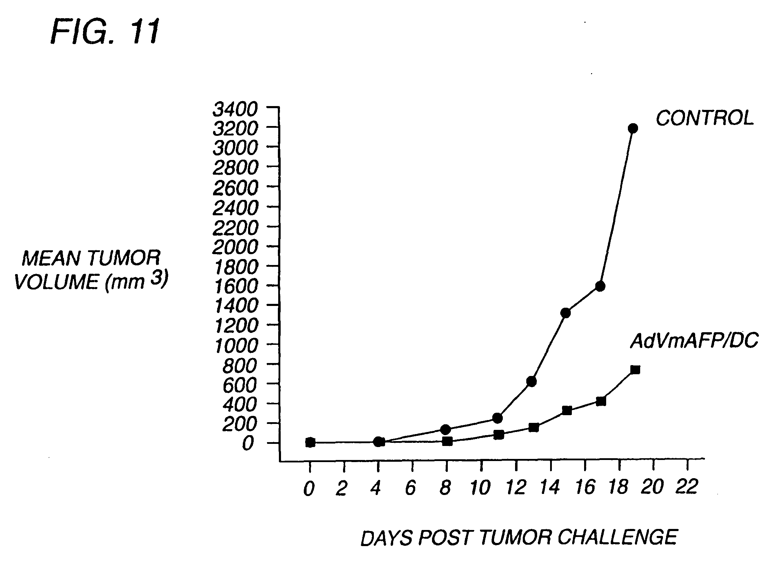

Further, another group of five mice was prepared by giving one iv injection per week for two weeks of 5x10⁵ dendritic cells transduced at an MOI of 100 with AdVmAFP. The response of this group to challenge with 4x10⁶ BWIC3 tumor cells one week after the last injection of transduced dendritic cells was compared to the response of a group of similar but uninjected control mice. The results of this test are shown in Figure 11. As can be seen, the immunized mice (closed squares) showed significant protection against the tumor challenge compared to the control mice (closed circles), demonstrating the effectiveness of the treatment with transduced dendritic cells.

Therefore, as can be appreciated from this disclosure, the present invention includes preventing or treating a cancer in a mammal, including a human, where the cancer cells bear at least part of the alphafetoprotein molecule as a surface marker. The prevention or treatment is accomplished by administering to the mammal a composition including immune system cells, such as dendritic cells, transduced with a recombinant vector that expresses alphafetoprotein cDNA.

### EXAMPLE I

### TREATMENT OF HEPATOCELLULAR CARCINOMA IN A MAMMAL

According to one embodiment of the present invention, there is provided a method for treating hepatocellular carcinoma in a human by creating an immune response in the human to at least part of the alphafetoprotein molecule. The method includes immunizing the human in a method similar to one of the methods disclosed herein or a corresponding method, or genetically manipulating the human to produce an immune response to alphafetoprotein. In a preferred embodiment, the human with hepatocellular carcinoma is immunized to produce an immune response to at least part of the human alphafetoprotein molecule, such as to AFP5, AFP7, AFP13, AFP14, AFP18, AFP22, AFP23, AFP28 AFP38, AFP39, AFP45 or AFP49. This immunization causes the human's immune system to attack the hepatocellular carcinoma cells having that portion of the alphafetoprotein molecule on their surface.

## Claims

1. A composition comprising a peptide consisting of part of an amino acid sequence of an alphafetoprotein molecule or a variant thereof, wherein the part of the alphafetoprotein molecule has an amino acid sequence selected from the group consisting of residues 1-9 of SEQ ID No:2, residues 12-20 of SEQ ID No:2, residues 158-166 of SEQ ID No:2, residues 178-186 of SEQ ID No:2, residues 235-243 of SEQ ID No:2, residues 287-295 of SEQ ID No:2, residues 404-412 of SEQ ID No:2, residues 441-450 of SEQ ID No:2, residues 492-500 of SEQ ID No:2, residues 542-550 of SEQ ID No:2, residues 547-556 of SEQ ID No:2, and residues 555-563 of SEQ ID No:2, and the variant has an amino acid sequence selected from the group consisting of SEQ ID NO:3 and SEQ ID NO:4.

2. The use of a peptide consisting of part of an amino acid sequence of an alphafetoprotein molecule or a variant thereof, wherein the part of the alphafetoprotein molecule has an amino acid sequence selected from the group consisting of residues 1-9 of SEQ ID No:2, residues 12-20 of SEQ ID No:2, residues 158-166 of SEQ ID No:2, residues 178-186 of SEQ ID No:2, residues 235-243 of SEQ ID No:2, residues 287-295 of SEQ ID No:2, residues 404-412 of SEQ ID No:2, residues 441-450 of SEQ ID No:2, residues 492-500 of SEQ ID No:2, residues 542-550 of SEQ ID No:2, residues 547-556 of SEQ ID No:2, and residues 555-563 of SEQ ID No:2, and the variant has an amino acid sequence selected from the group consisting of SEQ ID NO:3 and SEQ ID NO:4 for the manufacture of a medicament for treating cancer in a mammal by activating alphafetoprotein peptide specific T lymphocytes to create an immune response against cancer bearing these markers.

3. The use of a peptide as claimed in claim 2 wherein the cancer is hepatocellular carcinoma.

4. The use of a cDNA encoding a part of an amino acid sequence of an alphafetoprotein molecule or a variant thereof, wherein the part of the alphafetoprotein molecule has an amino acid sequence selected from the group consisting of residues 1-9 of SEQ ID No:2, residues 12-20 of SEQ ID No:2, residues 158-166 of SEQ ID No:2, residues 178-186 of SEQ ID No:2, residues 235-243 of SEQ ID No:2, residues 287-295 of SEQ ID No:2, residues 404-412 of SEQ ID No:2, residues 441-450 of SEQ ID No:2, residues 492-500 of SEQ ID No:2, residues 542-550 of SEQ ID No:2, residues 547-556 of SEQ ID No:2, and residues 555-563 of SEQ ID No:2, and the variant has an amino acid sequence selected from the group consisting of SEQ ID NO:3 and SEQ ID NO:4 for the manufacture of a medicament for treating cancer in a mammal by activating alphafetoprotein peptide specific T lymphocytes to create an immune response against cancer bearing these markers.

## Patentansprüche

1. Zusammensetzung, umfassend ein Peptid, bestehend aus einem Teil einer Aminosäuresequenz eines Alphafetoproteinmoleküls oder einer Variante davon, worin der Teil des Alphafetoproteinmoleküls eine Aminosäuresequenz aufweist, die aus der Gruppe ausgewählt ist, die aus folgendem besteht: Reste 1-9 von SEQ-ID-Nr. 2, Reste 12-20 von SEQ-ID-Nr. 2, Reste 158-166 von SEQ-ID-Nr. 2, Reste 178-186 von SEQ-ID-Nr. 2, Reste 235-243 von SEQ-ID-Nr. 2, Reste 287-295 von SEQ-ID-Nr. 2, Reste 404-412 von SEQ-ID-Nr. 2, Reste 441-450 von SEQ-ID-Nr. 2, Reste 492-500 von SEQ-ID-Nr. 2, Reste 542-550 von SEQ-ID-Nr. 2, Reste 547-556 von SEQ-ID-Nr. 2 und Reste 555-563 von SEQ-ID-Nr. 2, und die Variante eine Aminosäuresequenz aufweist, die aus der Gruppe ausgewählt ist, die aus SEQ-ID-Nr. 3 und SEQ-ID-Nr. 4 besteht.

2. Verwendung eines Peptids, bestehend aus einem Teil einer Aminosäuresequenz eines Alphafetoproteinmoleküls oder einer Variante davon, worin der Teil des Alphafetoproteinmoleküls eine Aminosäuresequenz aufweist, die aus der Gruppe ausgewählt ist, die aus folgendem besteht: Reste 1-9 von SEQ-ID-Nr. 2, Reste 12-20 von SEQ-ID-Nr. 2, Reste 158-166 von SEQ-ID-Nr. 2, Reste 178-186 von SEQ-ID-Nr. 2, Reste 235-243 von SEQ-ID-Nr. 2, Reste 287-295 von SEQ-ID-Nr. 2, Reste 404-412 von SEQ-ID-Nr. 2, Reste 441-450 von SEQ-ID-Nr. 2, Reste 492-500 von SEQ-ID-Nr. 2, Reste 542-550 von SEQ-ID-Nr. 2, Reste 547-556 von SEQ-ID-Nr. 2 und Reste 555-563 von SEQ-ID-Nr. 2, und die Variante eine Aminosäuresequenz aufweist, die aus der Gruppe ausgewählt ist, die aus SEQ-ID-Nr. 3 und SEQ-ID-Nr. 4 besteht, zur Herstellung eines Medikaments zur Behandlung von Krebs in einem Säugetier durch Aktivierung der Alphafetoproteinpeptid-spezifischen T-Lymphozyten, um eine Immunantwort gegen Krebs, der diese Marker aufweist, hervorzurufen.

3. Verwendung eines Peptids nach Anspruch 2, worin der Krebs ein hepatozelluläres Karzinom darstellt.

4. Verwendung einer cDNA, kodierend einen Teil einer Aminosäuresequenz eines Alphafetoproteinmoleküls oder einer Variante davon, worin der Teil des Alphafetoproteinmoleküls eine Aminosäuresequenz aufweist, die aus der Gruppe ausgewählt ist, die aus folgendem besteht: Reste 1-9 von SEQ-ID-Nr. 2, Reste 12-20 von SEQ-ID-Nr. 2, Reste 158-166 von SEQ-ID-Nr. 2, Reste 178-186 von SEQ-ID-Nr. 2, Reste 235-243 von SEQ-ID-Nr. 2, Reste 287-295 von SEQ-ID-Nr. 2, Reste 404-412 von SEQ-ID-Nr. 2, Reste 441-450 von SEQ-ID-Nr. 2, Reste 492-500 von SEQ-ID-Nr. 2, Reste 542-550 von SEQ-ID-Nr. 2, Reste 547-556 von SEQ-ID-Nr. 2 und Reste 555-563 von SEQ-ID-Nr. 2, und die Variante eine Aminosäuresequenz aufweist, die aus der Gruppe ausgewählt ist, die aus SEQ-ID-Nr. 3 und SEQ-ID-Nr. 4 besteht, zur Herstellung eines Medikaments zur Behandlung von Krebs in einem Säugetier durch Aktivierung der Alphafetoproteinpeptid-spezifischen T-Lymphozyten, um eine Immunantwort gegen Krebs, der diese Marker aufweist, hervorzurufen.

## Revendications

1. Composition comprenant un peptide se composant d'une partie d'une séquence d'acides aminés d'une molécule d'alphafetoprotéine ou d'une variante de cette partie, dans laquelle la partie de la molécule d'alphafetoprotéine a une séquence d'acides aminés choisie dans le groupe constitué par les résidus 1 à 9 de la SEQ ID N° : 2, les résidus 12 à 20 de la SEQ ID N° : 2, les résidus 158 à 166 de la SEQ ID N° : 2, les résidus 178 à 186 de la SEQ ID N° : 2, les résidus 235 à 243 de la SEQ ID N° : 2, les résidus 287 à 295 de la SEQ ID N° : 2, les résidus 404 à 412 de la SEQ ID N° : 2, les résidus 441 à 450 de la SEQ ID N° : 2, les résidus 492 à 500 de la SEQ ID N° : 2, les résidus 542 à 550 de la SEQ ID N° : 2, les résidus 547 à 556 de la SEQ ID N° : 2 et les résidus 555 à 563 de la SEQ ID N° : 2, et la variante a une séquence d'acides aminés choisie dans le groupe constitué par la SEQ ID N° : 3 et la SEQ ID N° : 4.

2. Utilisation d'un peptide se composant d'une partie d'une séquence d'acides aminés d'une molécule d'alphafetoprotéine ou d'une variante de cette partie, dans laquelle la partie de la molécule d'alphafetoprotéine a une séquence d'acides aminés choisie dans le groupe constitué par les résidus 1 à 9 de la SEQ ID N° : 2, les résidus 12 à 20 de la SEQ ID N° : 2, les résidus 158 à 166 de la SEQ ID N° : 2, les résidus 178 à 186 de la SEQ ID N° : 2, les résidus 235 à 243 de la SEQ ID N° : 2, les résidus 287 à 295 de la SEQ ID N° : 2, les résidus 404 à 412 de la SEQ ID N° : 2, les résidus 441 à 450 de la SEQ ID N° : 2, les résidus 492 à 500 de la SEQ ID N° : 2, les résidus 542 à 550 de la SEQ ID N° : 2, les résidus 547 à 556 de la SEQ ID N° : 2 et les résidus 555 à 563 de la SEQ ID N° : 2, et la variante a une séquence d'acides aminés choisie dans le groupe constitué par la SEQ ID N° : 3 et la SEQ ID N° : 4 pour la fabrication d'un médicament en vue de traiter un cancer chez un mammifère en activant les lymphocytes T spécifiques au peptide de l'alphafetoprotéine pour créer une réponse immunitaire contre le cancer portant ces marqueurs.

3. Utilisation d'un peptide selon la revendication 2, dans laquelle le cancer est un carcinome hépatocellulaire.

4. Utilisation d'un ADNc codant une partie d'une séquence d'acides aminés d'une molécule d'alphafetoprotéine ou une variante de cette partie, dans laquelle la partie de la molécule d'alphafetoprotéine a une séquence d'acides aminés choisie dans le groupe constitué par les résidus 1 à 9 de la SEQ ID N° : 2, les résidus 12 à 20 de la SEQ ID N° : 2, les résidus 158 à 166 de la SEQ ID N° : 2, les résidus 178 à 186 de la SEQ ID N° : 2, les résidus 235 à 243 de la SEQ ID N° : 2, les résidus 287 à 295 de la SEQ ID N° : 2, les résidus 404 à 412 de la SEQ ID N° : 2, les résidus 441 à 450 de la SEQ ID N° : 2, les résidus 492 à 500 de la SEQ ID N° : 2, les résidus 542 à 550 de la SEQ ID N° : 2, les résidus 547 à 556 de la SEQ ID N° : 2 et les résidus 555 à 563 de la SEQ ID N° : 2, et la variante a une séquence d'acides aminés choisie dans le groupe constitué par la SEQ ID N° : 3 et la SEQ ID N° : 4 pour la fabrication d'un médicament en vue de traiter un cancer chez un mammifère en activant les lymphocytes T spécifiques au peptide de l'alphafetoprotéine pour créer une réponse immunitaire contre le cancer portant ces marqueurs.
